(19) Europäisches Patentamt
European Patent Office
Office européen des brevets

(11) EP 0 620 863 B1

(12) **EUROPEAN PATENT SPECIFICATION**

(45) Date of publication and mention of the grant of the patent:
**03.05.2000 Bulletin 2000/18**

(21) Application number: **93900360.4**

(22) Date of filing: **23.12.1992**

(51) Int. Cl.[7]: **C12Q 1/68**, C07H 21/00, B01J 20/32

(86) International application number:
**PCT/GB92/02394**

(87) International publication number:
**WO 93/13220 (08.07.1993 Gazette 1993/16)**

(54) **MANIPULATING NUCLEIC ACID SEQUENCES**

MANIPULATION VON NUKLEINSÄURESEQUENZEN

MANIPULATION DE SEQUENCES D'ACIDES NUCLEIQUES

(84) Designated Contracting States:
**AT BE CH DE DK ES FR GB GR IE IT LI LU MC NL PT SE**

(30) Priority: **24.12.1991 GB 9127415**
**08.08.1992 GB 9216872**
**08.08.1992 GB 9216882**

(43) Date of publication of application:
**26.10.1994 Bulletin 1994/43**

(73) Proprietor:
**TEPNEL MEDICAL LIMITED**
**Manchester M1 4LF (GB)**

(72) Inventor:
**MINTER, Stephen**
**Moor Lodge Farm**
**New Mills SK12 4QL (GB)**

(74) Representative:
**Atkinson, Peter Birch et al**
**MARKS & CLERK,**
**Sussex House,**
**83-85 Mosley Street**
**Manchester M2 3LG (GB)**

(56) References cited:

**EP-A- 0 184 056** **EP-A- 0 356 838**
**EP-A- 0 455 905** **EP-A- 0 487 104**
**WO-A-86/07281** **WO-A-89/09282**
**WO-A-90/06042** **WO-A-90/09455**

- **Derwent WPI AN 89-232130 [32] & JP-A-1 168 286**
- **Declaration of Dr. Stephen Minter, dated 24.11.1997**
- **Declaration of Dr. Sandy Primrose, dated 03.12.1997**
- **A Dictionary of Genetic Engineering, S.G. Oliver & J.M. Ward, Cambridge University Press, 1988, page 31**

Remarks:

The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

The present invention relates to a method and apparatus for carrying out manipulations on nucleic acid sequences using a solid support system, as well as to supports for use in the method and apparatus.

Various procedures are known for manipulating nucleic acid sequences. For example, EP-A-0 200 362 (Cetus) describes a liquid phase process for amplifying, detecting and/or cloning nucleic acid sequences. The process comprises the following steps

(i) treating separate complementary strands of the nucleic acid sequence with two oligonucleotide primers each of which hybridises to one of the strands;

(ii) extending the primers to form double stranded nucleic acid sequences;

(iii) denaturing the product from (ii) to produce single strands of nucleic acid; and

(iv) using the single strands from (iii) in repeat of steps (i)-(iii) the overall procedure being repeated as often as necessary.

It is a feature of this prior technique that both complementary strands are used as templates for the second and further amplification steps. Furthermore in the method of EP-A-0 200 362 the reactant mixture includes unhybridised target nucleic acid, unhybridised copy target, and unhybridised primer which makes the system very inefficient. Additionally any mistake which occurs in the copying at any stage results in the mistake being copied into the "chain reaction".

Amplification techniques using solid phase support systems are also known, for example DYNAL (Trade Mark) magnetic beads as disclosed in EP-A-0 091 453 and EP-A-0 106 873. In use of such beads, DNA is synthesised onto a magnetic bead and then cleaved therefrom by ammonium hydroxide. The beads may then be separated from the synthesised DNA using a magnet. Alternatively, biotin may be added to one end of a synthesised or natural DNA sequence and the sequence recovered using a magnetic bead which has been pre-conjugated to streptavidin (thereby attracting the biotin to recover the DNA). A problem with this type of solid support is that the biotin streptavidin linkage is biodegradable.

Other known solid support systems include porous silicas. The presence of the pores can create problems since nucleotide chain growth occurs within the pores, resulting in inefficient washing and residues remaining within the pores, again reducing the yield and resulting in relatively inefficient coupling.

EP-A-0 184 056 describes a method for the large scale production of DNA probes using a solid substrate. The process of this prior specification comprises covalently linking to a solid substrate a polynucleotide complementary to the probe to be produced and then hybridising the polynucleotide with an oligonucleotide which acts as a primer. The oligonucleotide is then extended in a direction away from the substrate (using the polynucleotide as a template) thereby to produce an extended sequence complementary to the bound polynucleotide. The hybridised polynucleotide and extended oligonucleotide are then denatured so as to release the extended oligonucleotide from the solid substrate for collection. The extended oligonucleotide may be used as an analytical probe. Thus, for example, the polynucleotide originally bound to the support may be a gene, and the extended oligonucleotide may be used as a probe for detecting the presence of that gene in a biological sample.

There are however a number of disadvantages associated with the method described in EP-A-0 184 056. In particular, if the polynucleotide to be bound to the support is not "pure" and contains other polynucleotides then these will also become bound to the support. As a result, the oligonucleotide may also hybridise to these other polynucleotides so that the extended oligonucleotide ultimately obtained may in fact be a mixture of products. Therefore, the method is not particularly good for producing "pure" samples of nucleic acid.

It is therefore an object of the present invention to obviate or mitigate the abovementioned disadvantages.

According to a first aspect of the present invention there is provided a method of effecting a manipulation of a nucleic acid sequence comprising the steps of

(a) providing in a flow-through vessel a solid support system having covalently bonded thereto, single stranded oligonucleotide having a sequence of bases complementary to a portion of the sequence of a particular target nucleic acid having a greater number of bases than said oligonucleotide, said oligonucleotides being specific for said target nucleic acid,

(b) adding a source of single stranded target nucleic acid to the solid support system,

(c) hybridising the target nucleic acid to the oligonucleotide,

(d) effecting a flow through washing of the support after said hybridisation to remove any non-hybridised target nucleic acid in said support system thereby providing a pure sample of the target nucleic acid on the solid support system, and

(e) effecting on the target nucleic acid immobilised on the support in the flow through vessel a manipulation selected from copying of, denaturation of and/or hybridisation to said target nucleic acid.

The above method results in the target nucleic acid being selectively hybridised to the oligonucleotide on the support. Any impurities which are present in the system (e.g. as introduced in the sample containing the target nucleic acid) may be removed by washing to leave a "clean" sample of target nucleic acid on which the manipulation may be effected. Washing will be effected at a temperature at which the target nucleic acid does not melt off the oligonucleotide

The target nucleic acid may for example be a purified or non-purified, native or synthesised nucleic acid. The target may be any DNA or RNA sequence from a viral, bacterial, animal or plant source.

The oligonucleotide bound to the support will generally comprise at least 8 nucleotides. Typically the polynucleotide to be hybridised thereto will comprise 1000-2000 bases and will be significantly longer than the oligonucleotide.

The oligonucleotide may be bonded to the support by reaction between suitable reactive groups provided on the support and the oligonucleotide. Alternatively the oligonucleotide may be synthesised in situ on the support.

The oligonucleotide may be bound to the solid support in the 3'-5' or the 5'-3' orientation.

Any protecting groups on the oligonucleotide are removed before the hybridisation step (c).

In the method of the invention, the single stranded target nucleic acid is hybridised to the oligonucleotide bound to the support. The oligonucleotide sequence will be very specific for the target to be hybridised. As a result, the hybridisation may be effected at a temperature which is very specific for hybridisation between the oligonucleotide and target. The hybridisation of the target nucleic acid to the oligonucleotide on the support will ideally be effected at a temperature just below (e.g. 1-3 ° C below) the temperature ($T_m$) at which the target nucleic acid will "melt off" the oligonucleotide. As a general rule, $T_m$ may be calculated from the following guideline formula known in the art

$$T_m = 2 (A + T) + 3 (G + C)$$

where A, T, G and C are respectively the number of adenine. thymine, guanine and cytosine residues present in the oligonucleotide bound to the support. The formula is usually applicable for sequences of up to about 30 nucleotides. After the hybridisation step, the support may be washed at a temperature slightly lower than $T_m$ thereby removing any unannealed target and impurities (e.g. proteins or unwanted nucleic acid sequences). Thus it is possible to add to the solid support system a mixture of polynucleotides (such as may be present in a biological sample) but only the specific polynucleotide of interest (i.e. the target) is retained on the support. Thus, after washing, manipulations of the polynucleotide may be effected on a "pure"
sample thereof.

Successive manipulations may be carried out on the hybridised target nucleic acid. Alternatively an initial manipulation may be effected to produce a copy of the target bound to the support. This copy may then be used for subsequent manipulation operations.

Between each manipulation the support may be washed as necessary to remove impurities and product may then be collected as required. After further washing of the support (if necessary) the manipulation may be repeated.

The manipulation may comprise for example copying, amplification of the target nucleic acid sequence, in vitro transcription, or purification of DNA binding proteins. The manipulation may also be for detecting the presence of target by testing for hybridisation of a labelled primer to the target.

Manipulations on the polynucleotide sequence may be effected using procedures known in the art. For example, DNA polymerase I, Klenow fragments thereof, thermostable polymerase or reverse transcriptase along with deoxynucleotide triphosphates and/or dideoxynucleotide triphosphates may be used as appropriate in the manipulation processes (described below). The nucleotides may if desired be labelled with, for example, $^{35}S$, $^{32}P$, chromophores, biotin, or any other biological marker of choice.

The method of the invention is particularly useful for the analysis of samples (eg medical samples) to detect the presence (or otherwise) of a particular nucleic acid. If, for example, the sample is one being tested for a medical condition in which a relatively high amount of a particular nucleic acid (the target) is present then detection of the target nucleic acid may be effected as follows. After the target (if present) has been hybridised to the support and the latter washed to leave a "clean" sample of target, a labelled oligonucleotide primer which is known to be complementary to a

sequence on the target is added to the support under conditions in which the primer will hybridise to the target. The label may, for example, be a radioactive label, a chromophore, or enzyme linked reactant. The support is then washed to remove unannealed primer, the washing being effected at a temperature below that at which primer hybridised to target nucleic acid is "melted off" the target. Subsequently the temperature of the support may be raised to melt off hybridised primer (either with or without target DNA). A detector may then be used to detect the presence of primer. If primer is detected then this is confirmation that the target nucleic acid was present in the sample.

If however the sample is one which is likely to contain only a relatively low amount of the target nucleic acid then repeated amplification reactions (see below) may be effected (each producing labelled copies of amplified product if the originally suspected target was present). A detection operation may then be effected to determine whether labelled amplification product has been produced. If so, this is confirmation that the originally suspected target was present. It will of course be appreciated that any desired number of amplifications may be performed so that the test may be made very sensitive in terms of being able to determine the presence of very small amounts of target in the original sample.

It is a significant feature of the invention that detection of the presence of target in the original sample may be achieved without the need to use separation of products on gels.

In accordance with the invention the support is provided in a flow-through vessel (e.g. a column) which facilitates the washing of the support as well as the subsequent manipulation as will be appreciated from the subsequent description.

The use of a flow-through vessel is an important feature and therefore according to a second aspect of the present invention there is provided a flow through vessel having an inlet and an outlet and containing a solid support system having covalently bonded thereto, by base stable linkages, single stranded oligonucleotide having a sequence of bases complementary to a portion of the sequence of a particular target nucleic acid having a greater number of bases than said oligonucleotide, said oligonucleotide being specific for said target nucleic acid, said vessel having porous retaining elements to retain said particles in the vessel.

The vessel may for example have a size of 150-250 µl.

The manipulation may be effected using an apparatus (in which the flow through vessel is located) for supplying reactant solutions to the vessel and for collecting and detecting the product of the manipulation.

According to a third aspect of the present invention there is provided apparatus for effecting a manipulation on a nucleic acid sequence comprising

a flow through vessel provided with a solid support system to which are covalently bonded, by base stable linkages, single stranded oligonucleotide having a sequence of bases complementary to a portion of the sequence of a particular target nucleic acid having a greater number of bases than said oligonucleotide, said oligonucleotide being specific for said target nucleic acid,

storage means for storing solutions removed from the vessel during washing or other procedures before the solutions are returned to the vessel,

detector means for detecting products of nucleic acid manipulations, and control means for diverting solutions into and out of the vessel, into waste or storage areas and to the detector means.

In a preferred form of the apparatus, the outlet of the vessel may selectively communicate with (i) a reagent storage region, (ii) a manipulation product detection region, and (iii) a waste outlet. The reagent collection region allows reagent solution initially supplied to the vessel <u>via</u> the inlet thereof to be passed to the reagent collection region for subsequent return to the vessel where repeated manipulations on the same target nucleic acid are (or copy thereof) to be effected. Product from each manipulation operation may then be passed to the detection region for detection purposes. If desired, the apparatus may be such that product from successive manipulations is collected prior to being passed to the detector.

The apparatus will of course include a suitable valving arrangement (e.g. solenoid operated valves) to permit solutions, product etc. to be moved (preferably under gas pressure) through the apparatus.

The solid support may comprise non-porous particles having a size of 100 to 200 µm (microns). The use of a non-porous material is particularly advantageous since it avoids certain problems associated with porous supports used in the prior art, namely nucleotide chain growth occurring within the pores resulting in inefficient washing and residues remaining within the pores, again reducing yield and resulting in relatively inefficient coupling. The support may be of calcined spherical particles of diameter 100 to 200 µm (microns). The support may for example be non-porous silica gel.

The support (prior to immobilisation of the oligonucleotides) may have reactive groups (e.g. epoxy groups) for use in immobilising the oligonucleotide sequence on the supports.

It is preferred that the support has a cross-linked siloxane matrix (prior to immobilisation of the oligonucleotides) having reactive groups which may be used for providing an immobilised oligonucleotide on the support.

The use of the siloxane matrix is particularly advantageous due to its acid/base stability This is in contrast to prior art supports incorporating a biotin streptavidin linkage which is biodegradable. The siloxane matrix allows a wide range of manipulations to be carried out without the oligonucleotide being removed from the support. For example, ammonia may be used as a reagent for deprotecting the oligonucleotide which remains on the support. Normal succinic acid linkages as used for immobilising oligonucleotide are base labile, causing the oligonucleotide to leave the support.

Preferably the reactive group which may be used for the immobilisation of the nucleic acid sequence is an epoxy group.

If desired, free hydroxyl groups present on the support after reaction with the siloxane matrix precursor but before formation of the cross-linked siloxane matrix may be capped e.g. by using a chlorosilane.

Preferably the siloxane matrix precursor is a glycidoxy compound of the formula

$$\begin{array}{l} (RO)_3 - Si - R^1 - O - CH_2 - CH - CH_2 \\ \qquad\qquad\qquad\qquad\qquad\qquad\quad \backslash \quad / \\ \qquad\qquad\qquad\qquad\qquad\qquad\qquad O \end{array}$$

where R is an alkyl group of 1 to 4 carbon atoms and R' is an alkylene residue. Most preferably R is methyl and R' is $-(CH_2)_3-$.

A synthetic oligonucleotide may be covalently bound to the support via the epoxy group. Alternatively, a sodium salt of a nucleotide may be bound covalently to the support and oligonucleotide synthesis (using β-cyanoethyl phosphoamidite) may be conducted.

The invention will be further described by way of example only with reference to the accompanying drawings in which:

| | |
|---|---|
| Figure 1 | diagrammatically represents the principle of the invention; |
| Figure 2 | shows a longitudinal section of a flow through column containing the oligo-solid system; |
| Figure 3 | illustrates a reaction path for obtaining a solid support for immobilising an oligonucleotide; |
| Figures 4a and 4b | show a diagrammatic representation of the solid support system and reactions involved in binding an oligonucleotide thereto: |
| Figure 5a-5d | shows diagrammatic representations of the steps involved in amplification of a target nucleic acid; |
| Figure 6 | illustrates an apparatus for effecting a manipulation in accordance with the invention; and |
| Figure 7 | is an autoradiograph showing the results of Example 3. |

Referring to Fig. 1, there are illustrated (to a much enlarged scale) a plurality of particles 1 of a solid support material each having immobilised thereon a number of identical oligonucleotide sequences 2 which are specific for a single stranded target nucleic acid sequence 3 contained within a sample 4 which also includes unwanted impurities (e.g. other nucleic acid sequences, proteins etc.) as represented by the triangles 5.

Sample 4 is added to the support 1 under hybridising conditions such that some of the nucleic acid sequences 3 bind to the oligonucleotide leaving non-hybridised acid 3 together with the impurities 5 in the liquid phase. During subsequent washing, the non-hybridised acid 3 together with impurities 5 are removed from the support 1 as shown to leave a clean sample of target nucleic acid 3 bound to the support.

It is particularly preferred that the particles 1 are contained within a flow through column 6 (see Fig. 2) having inlet and outlet valves 7 and 8 as shown as well as a heater 9. The particles may be retained within the column by porous elements 10. For convenience, the particles 1 depicted in Fig. 2 are shown as having only one oligonucleotide sequence immobilised thereon.

The target nucleic acid is added to the flow through column via inlet valve 7 in a ratio of for example 1000:1 oligonucleotide:target in suitable buffer, e.g. high salt hybridisation buffer and hybridisation effected under conditions specific for the support bound oligonucleotide and target nucleic acid. When double stranded DNA is the source of the target nucleic acid, it is first separated into single strands by conventional methods either before being added to the column or whilst it is on the column.

Sample 4 is retained in the column by virtue of the outlet valve 8 being closed. After the hybridisation step, the unwanted impurities 5 (which are not immobilised on the support) may be removed by washing (with valve 8 open) to leave the clean sample target nucleic acid on the support.

The manner in which the solid support (with immobilised oligonucleotide) is prepared is depicted in Figs. 3-5. Reference is firstly made to Fig. 3 which illustrates one embodiment of reaction path for obtaining a support with reactive groups to which an oligonucleotide may be immobilised.

The procedure starts with silica gel particles (I) having surface hydroxyl groups as shown. In the first reaction step, the particles are treated with 3-glycidoxypropyl trimethoxy silane (II) e.g. at a temperature not in excess of 95° C for 2 hours under a nitrogen atmosphere. As a result, a condensation reaction occurs whereby residues of (II) become bonded to the silica particle (I) to yield the product depicted as (III). Although the drawing shows only two residues bonded to the silica particle this is simply for the purpose of clarity and in practice considerably more of the residues will be bonded to the silica particle. Typically, the level of binding will be about 40-45 micromoles of (I)/gram of silica.

Product (III) is then washed in sequence with dry toluene, dry methanol, and dry ether.

The next step involves heating of the product to effect a cross-linking of the residues. Typically this cross-linking reaction is effected at a temperature of 110° C for at least 2 hours. The resultant product is as shown at (IV) from which it will be seen that the silicon atoms are linked together through oxygen atoms.

Free hydroxyl groups remaining on the surface of the silica particle may if desired be capped with a chlorosilane. The capping agent may, for example be trimethyl chlorosilane with the reaction being conducted in pyridine for two hours at room temperature. Subsequent to capping, the support may be subjected to the above described washing operation.

The support thus obtained has free epoxy groups which may be used for immobilisation of an oligonucleotide.

It is possible for example using known procedures to synthesise an oligonucleotide in situ on the support, the oligonucleotide being complementary to a specific sequence on a target nucleic acid. Thus, the sodium salt of a dimethoxy trityl (DMT) protected nucleotide may be reacted with the support whereby the $-O^-$ moiety at the 3' position of the nucleotide reacts with the free epoxy group to yield product (V). The sodium salt may be prepared from the DMT nucleotide (which has been dried over $P_2O_5$) by dissolving in dry DMF, maintaining the solution under an anhydrous atmosphere, and adding sodium hydride. Subsequently the sodium hydride is filtered off to leave the sodium salt.

After removal of the DMT protecting group, a desired oligonucleotide can be synthesised from the individual nucleotides using known procedures for oligonucleotide synthesis.

Alternatively it is possible for a pre-synthesised oligonucleotide (complementary to a specific sequence on a target nucleic acid and preferably also containing a restriction endonuclease site) to be bound to the support.

A number of synthetic oligonucleotides are available complementary to a number of target nucleic acid sequences which may be used as probes to test for the presence of certain bacteria, viruses, parasites and the like, see appendix A. By way of specific example, the invention may be used, for example, for detecting the following, for which the oligonucleotide sequences given below would be immobilised on the support.

| | |
|---|---|
| Epsilon Bar Virus | GAC AAC TCG GCC GTG ATG GA |
| Toxoplasma gondii | GGA ACT GCA TCC GTT CAT GAG |
| Trypanosoma brucei brucei | CGA ATG AAT ATT AAA CAA TGC GCA G |

The oligonucleotide may be bound to the support in either the 3'-5' or 5'-3' orientation. Binding in the 3'-5' orientation may be achieved by reacting the epoxy group on the support with a primary hydroxyl group of the sodium salt of a nucleotide (e.g. thymidine nucleotide (dimethoxytrityl deoxyribonucleoside (dT) (- see Fig. 4a)) to produce a bond which is both acid and alkali stable, unlike currently used linkage groups.

Binding in the 5'-3' orientation may be achieved as follows (see also Fig. 4b) 5'-iodo 5'-deoxythymidine is reacted with sodium triphenyl methylmercaptile in DMF to form an S-trityl compound. This compound is further reacted with diisopropylammo tetrazolide and 2-cyanoethoxy bis (NN diisopropylamino) phosphoamidite in DCM to produce the β cyanoethyl. The S-trityl group is removed by reducing reactions by methods known in the art (Connolly, Nucleic Acid Research, 13, 12, 1985; Chu, Nucleic Acid Research, 16, 9, 1988; Guar, Nucleic Acid Research, 17, 11, 1989) before linking with the epoxy substituted support using sodium hydride. Other methods of preparing oligonucleotides for linkage in the 5'-3' orientation are known (Anisorge, Nucleic Acid Research, 15, 11, 1987; Sproat, Nucleic Acid Research, 15, 12, 1987; Zuckerman, Nucleic Acid Research, 13, 5305, 1987; Verheyden, JOREGA CHEM, 35, 2319, 1970), although this list is not exhaustive and any other method apparent to those skilled in the art may be used to prepare oligonucleotides in either the 3'-5' orientation of 5'-3' orientation for linkage to the solid support system of the present invention.

Where the oligonucleotide has protecting groups due to the synthesis reaction, such as butyl/isopropyl groups on the bases (G,A,C) of the 3'-5' oligos and β cyanoethyl groups of the 5'-3' oligos, these must be removed after synthesis. The oligo-solid support may be heated to 55° C for 2 hours in 38% $NH_4OH$ to eliminate the protecting groups to give the deprotected oligonucleotide.

An example of a manipulation suitable for this system is amplification of the target nucleic acid sequence.

Figures 5a-c shows diagrammatically the steps involved in amplification techniques depending on the position of the primer site, orientation of the oligonucleotide (i.e. 3'-5' or 5'-3'), and therefore target nucleic acid sequence, and presence of one or two oligonucleotide sequences (i.e. reverse orientation oligos). The steps involved in amplification are known, i.e. addition of primer, primer extension, addition of a second primer to back copy the target copy, etc.

In the embodiment illustrated in Fig. 5a, the 5' end of the oligonucleotide may be bound to the support and the manipulation may be amplification of the target nucleic acid which is hybridised to the oligonucleotide. In this case, the oligonucleotide may serve as the primer or a separate primer 11 may be ligated to the oligonucleotide prior to the hybridisation step. In either case a primer extension product 12 is synthesised (using the target nucleic acid as a template) from the primer to the end of the target nucleic acid. There is thus obtained a nucleic acid sequence which is bound to the support and which is complementary to the target nucleic acid. For convenience, the complementary sequence is referred to herein as the "copy target".

The support may then be washed and the hybridised nucleic acid strands (i.e. the target and copy target) may subsequently be denatured using techniques well known in the art (e.g. by heating at a particular temperature) to leave only the copy target bound to the solid support (via the oligonucleotide). For convenience, the solid support with bound oligonucleotide is also referred to in the subsequent description as the -"oligo-solid support".

The support system may be washed and the copy target which remains thereon may then be used for synthesising further quantities of the original target nucleic acid. Such synthesis will comprise hybridising a primer 13 to the copy target, washing the support to remove unhybridised primer, effecting primer extension (whereby the copy target serves as a template), and then denaturing and collecting the synthesised nucleic acid sequence 14. This process may be repeated as many times as necessary whereby the original target nucleic acid is, in effect, amplified to a desired degree. It will be appreciated that each amplification step uses the same set of copy target molecules.

The procedure illustrated in Fig. 5a is particularly suitable for detecting the presence of low quantities of a target nucleic acid in a medical sample. The primer 13 may be labelled by known techniques and a detection operation is performed to detect the correspondingly labelled nucleic acid sequence 14. If the label is detected then this is confirmation that the target nucleic acid 3 was present in the original sample.

As a modification of the procedure described for Fig. 5a, the double stranded nucleic acid may include a restriction site (e.g. as provided by a primer ligated to the oligonucleotide) and the appropriate restriction enzyme then used to cleave the double stranded molecule from the support.

In an alternative embodiment of the invention (see Fig. 5b), in which the 3' end of the oligonucleotide is bound to the support, the manipulation may involve sequencing of the hybridised target nucleic acid using standard methodology (e.g. Sanger DNA sequencing technique). Thus, a primer 15 may be hybridised to the target nucleic acid (which is hybridised to the oligonucleotide on the support). The primer must be one which will hybridise to the target at a temperature at which the latter does not melt off the oligonucleotide. A strand synthesis reaction is then effected with a mixture of the 4 deoxynucleotides (dATP, dTTP, dGTP, dCTP) together with a single dideoxynucleotide. The synthesis proceeds in the 5'-3' direction from the primer towards the oligonucleotide. The reaction is carried out four times, using a different dideoxynucleotide each time. As is well known, the presence of the dideoxynucleotide causes nucleic acid sequences of different lengths to be obtained - as represented by the dashed lines. The products of the four reactions are run in parallel on an appropriate gel and the sequence determined from the positions of the bands in the four tracks. This particular embodiment of the invention is particularly suitable for detecting point mutations in a nucleic acid (provided, for example, as a medical sample) by comparing the sequence obtained with that of a control sample known to have the "normal" sequence.

The embodiment in which the 3' end of the oligonucleotide is bound to the support may also be used to amplify the target nucleic acid sequence. As in the case of sequencing described above, a primer may be hybridised to the target nucleic acid. The primer used must be one which hybridises to the acid at a temperature below that at which the target melts off the oligonucleotide. The extension of the primer is then effected under known conditions, the extension being effected towards, and as far as, the oligonucleotide. The copy product thus obtained may be melted off the target nucleic acid under conditions which leave the latter hybridised to the support for use in further amplification reactions.

In all of the amplification reactions described above, the same template remains bound to the oligo solid support and is used for further amplification reactions. This is a significant difference of the present invention over the technique disclosed in EP-A-0 200 362 since, in the latter, both strands of the target nucleic acid are used as templates and, once copied, the target strands and the copied strands are all used as templates for the second and further amplification steps. Thus should a mistake in the copying occur at any stage, the mistake is copied into the "chain reaction".

The present invention avoids this problem by either having a single copying stage and maintaining this copy of all future amplifications, or by maintaining the original target sequence for all amplifications. The present invention is also more efficient than that described in EP-A-0 200 362 since in the former non-hybridised primers and target or copy sequences are removed from the system by washing whereas, in the latter, unhybridised target, primer, and copy product are present in the method thus making the system inefficient.

In a still further embodiment (see Fig. 5c), two primers may be added which are complementary to different

portions of the target or copy target, in either the 5'-3' or 3'-5' orientations, and which may be used to check the sequence for point mutations in a manner known in the art (DNA ligase annealing reaction). In this analysis, the primers are usually labelled and the test used for rapid diagnosis of, for example, P53 tumour suppressor gene mutations since 75% of all colon cancer patients have a point deletion in this gene.

It will be appreciated from the above description that the present process differs from known processes in that the solid phase system and apparatus of the present invention allow greater efficiency and yield of the amplified nucleic acid. This is due to the ability of the process to be carried out in steps. The column may be washed after each hybridisation to eliminate unhybridised DNA etc and "clean up" the system, greatly improving efficiency. The contents of the reaction solution on the column, i.e. newly amplified target sequence, may be diverted directly into a detector such as an optical cell connected to the column and the presence or absence of a target sequence confirmed quickly. This is particularly applicable to diagnosis of patient samples to test for the presence of any disease-causing organism in a simple, quick and reliable manner not hitherto available.

A further embodiment of the invention (which does not involve amplification) is illustrated in Fig. 5d which is applicable to the testing of samples for the presence (or otherwise) of a relatively large amount of a target nucleic acid. In the embodiment of Fig. 5d, a labelled primer 16 may be added to the support system, the primer being one which will hybridise to the target nucleic acid at a temperature below that at which the target melts off the oligonucleotide. If target nucleic acid 3 is present, the primer will hybridise thereto. The support may now be washed at a temperature below that at which the primer 16 melts off the target nucleic acid to remove unannealed primer 16. In the next stage, the solid support is heated to a temperature at which primer 16 is melted off (either with or without target acid), the support is washed, and eluted product passed to a detector. If the label is detected then this demonstrates that presence of the target nucleic acid in the original sample.

Referring now to Fig. 6, there is illustrated an apparatus in which the hybridisation of the target nucleic acid to the support bound oligonucleotide as well as amplification reacts may be effected.

The apparatus illustrated in Fig. 6 comprises a column 20 (equivalent to column 6 of Fig. 2) pre-loaded with support particles 21 having oligonucleotide 22 bound thereto. Column 20 will typically have a volume of about 200 µl and has an inlet 23 and an outlet 24 provided with porous retaining elements 25 to maintain the particles 21 within the column. The apparatus has an arrangement of valves 26-34 as shown and is also provided with a source of pressurised gas which may be applied in accordance with arrows G. The gas is used for effecting movement of reagents and products within the apparatus. Certain of the valves are arranged to provide venting of the gas as depicted by arrows V.

Valve 26 allows selective communication of the interior of column 20 with a reagent supply assembly 35 having individual vessels 35a-35d containing solutions (e.g. samples, primers, buffers etc) which are supplied to the column 20 via open valves 26 and 27, by means of gas pressure. Valve 29 may be used during this procedure to vent excess gas pressure.

On the outlet side of valve 28 is a transfer region 36 which may selectively communicate (via valve 30) with a product collection region 37 associated with a detector 38, which may, for example, be an optical detector, or a detector for a radiolabel. Other types of detectors may also be used.

Transfer region 36 may also selectively communicate (via valve 31) with a solution holding region 39 or a waste receptacle 40. The solution collection region 39 is provided by one of the tubular lines of the apparatus and has a volume at least equal to that of column 20.

A heater 41 is provided around the column as shown.

The valves of the illustrated apparatus may for example be zero dead Teflon seated valves (e.g. as available from General Valve (U.S.A.). the tubing used in the apparatus may be 1.5 mm diameter Teflon tubing.

In use of the apparatus, the sample together with appropriate buffers is supplied to column 20 by gas pressure as previously indicated. Hybridisation of target nucleic acid is effected at the appropriate temperature and subsequently the column is washed (with solution supplied from assembly 35). The washings may be passed to waste receptacle 40 (via appropriately set valves 28 and 31). Valve 34 may be open during this procedure to vent excess pressure.

A manipulation may now be carried out on the target nucleic acid held on the support. For this purpose reagent solutions as appropriate are supplied from assembly 35 via appropriately set valves (both for liquid supply and for venting).

After the manipulation, the reagent solution may be passed from column 20 to solution holding region 39.

Manipulation product may now be melted off the support 21 and passed to product collection region 37 either for immediate detection or for storage.

If a further manipulation is to be effected on target nucleic acid (or copy thereof) on the support then the solution held in region 39 may be returned to column 20 under gas pressure through valve 32. The above process may then be repeated.

Manipulation product may be collected as often as required in region 37. During collection of product, valve 33 will be set to vent. To prevent product being passed immediately to the detector, valve 33 may be closed after a short

time so that collected product cannot pass sufficiently far along region 37 to reach detector 38. The provision of the gas supply to detector 38 allows a means of cleaning the detector when required.

It will be appreciated that the above described apparatus may be automated with the setting of the valves under electronic control.

The apparatus may comprise a single column as illustrated in Fig. 6 or may comprise a plurality of columns for testing a plurality of oligonucleotide probes on a single sample, or for testing a single oligonucleotide probe (e.g. HIV I) on a plurality of samples.

Additionally, column 20 need not be a fixed part of the apparatus. It is possible for example, to provide disposable columns 20 pre-loaded with solid support having oligonucleotide bound thereto, and then to locate the column in an apparatus otherwise as shown in Fig. 6. This avoids the need, which would be required for a fixed column, of emptying solid support from column.

The invention will be described by the following non-limiting Examples. The oligonucleotides used in the Examples - both column bound and column cleaved (primer samples) were synthesised by a standard coupling programme on a Biosearch Model 8500 DNA synthesizer. β-cyanoethyl protected phosphoamidites were utilised for the synthesis. The column cleaved primer samples were fully deblocked (all DMTr groups removed) and were automatically $NH_4OH$ cleaved from the CPG columns. The column bound oligonucleotides were not $NH_4OH$ cleaved and the final DMTr group was not removed unless stated. Following synthesis both column bound and column cleaved oligonucleotides were transferred to screw topped tubes with 38% $NH_4OH$ (1 ml). The tubes were placed, in clamps, for 1 hour in a 55° C oven, to remove protecting groups. The tubes and clamps were then transferred to a -20° C freezer for 10 minutes. The $NH_4OH$ supernatant was removed from the column bound oligonucleotides and discarded. The samples were freeze dried in a Savant centrifugal drier and stored in a desiccator. Each column cleaved oligonucleotide was divided into three tubes and freeze dried. One of these tubes was then taken and the crude oligonucleotide present purified.

<u>Example 1</u>

Preparation of Silica Gel Support.

Calcined Spherisorb GC support (1g) was taken and dried over $P_2O_5$ round bottomed flask with 40 ml of anhydrous toluene. The contents of the flask were stirred with a magnetic stirrer and the resulting slurry heated to 90-95° C in an oil bath. Anhydrous 3-glycidoxypropyl trimethoxy silane (1.5 ml) was added to the slurry and the reaction allowed to proceed, with stirring, for three hours at 90-95° C. Care was taken to ensure that the temperature did not exceed 95° C. After three hours the reaction mixture was cooled to room temperature, filtered, and washed with anhydrous toluene (40 ml), methanol (40 ml), and ether (20 ml). The functionalized silica gel was dried over $P_2O_5$ and silica overnight.

<u>Example 2</u>

Synthesis of Cytosine nucleotide substituted support

(a) Preparation of the sodium salt of the C nucleotide derivative: Dimethoxytrityl deoxyribonucleoside cytosine (DMTr dC) (1g) was taken and dried over $P_2O_5$ and silica for several days. The dried DMTr dC was dissolved in anhydrous N,N-dimethylformamide (DMF) (20 ml) under nitrogen. After solution was complete, sodium hydride (0.6 g) was added and allowed to react with the DMTr dC in anhydrous AMF for 20 minutes with stirring. The NaH was then filtered off from the reaction mixture.

(b) Reaction of the prepared silica gel and nucleoside derivative: The dried silica gel support was added directly to the filtered sodium salt of the nucleoside and the mixture stirred, under reflux, for two hours. The coupled nucleoside and support mixture was cooled to room temperature overnight. The substituted silica support was filtered through a silonised sintered glass filter and washed with 20 ml each of anhydrous DMF, anhydrous toluene, methanol, methanol and ether. Finally, the solid support was dried and stored in a desiccator.

The 5' oxygen of the sugar portion of the nucleoside derivative is protected by a DMTr group, this is acid labile and can be removed by anhydrous acids such as dichloroacetic acid (DCA), generating a bright orange DMTr cation. A portion of the dried substituted support was treated with dichloroacetic acid (1 ml of 2% DCA in dichloromethane) and a bright orange colour was seen, indicating that the coupling of the support silica and cytosine nucleoside derivative had been successful.

Example 3

As a test system a procedure was set up to isolate a clone from a mixture of bacterial and viral DNA. The target sequence (cloned gene) was located within the bacteriophage M13 mp and contained the following two sequences

GCG GGT CCC AAAA GGG TCA GTG CTG (1)

AGT GTG TCC TTT GTC GAT ACTG (2)

M13 mp is a standard virus used in molecular biology and is used routinely as a sequencing vehicle.

An oligonucleotide of the following sequence, i.e. homologous to sequence (1), was synthesised onto the support produced in Example 2.

CGC CCA GGG TTT CCC AGT CAC GAC (3)

The cytosine residue at the left hand end of the above sequence is that which was incorporated in the support produced in Example 2.

The solid support (15 mg) with oligonucleotide sequence bound thereto was then placed in a flow through column and a mixture of E. coli (product of $10^8$ cells) and 1 µg of M13 DNA was placed onto the column after being denatured by heat. The column was maintained at a temperature of ($T_m$-2)°C where Tm is the melting temperature of sequence (3) as calculated by the formula

$$T_m = 2 (A + T) + 3 (G + C)$$

where A, T, G and C are respectively the number of Adenine, Thymine, Guanine and Cytosine residues in sequence (3).

Since the column is maintained slightly below the melting temperature ($T_m$) the viral DNA is able to anneal to the bound oligonucleotide.

The column was maintained at ($T_m$-2)° C and washed with 2½ millilitres of annealing buffer (10 mM $MgCl_2$ and 10 mM TRIS HCl pH 7.5). The washings from the column were collected in 200 µl fractions (11 in total) in tubes.

Each of the collected fractions was then subject to a standard sequencing reaction (Sanger Coulson method) by addition of 1 ng of sequence (3) above to each of the tubes together with DNA polymerase (Klenow fragment) and deoxyribonucleotides and dideoxyribonucleotides in the standard reaction mixture. $^{32}P$ labelled deoxythymidine was also added to the reaction mixture.

A sequence reaction was also performed on the material immobilised on the column. The column bound material was melted off the column and collected. Sequencing was performed using the same reaction mixture as that employed in the solution phase sequencing.

The products of the sequence reactions, both support bound and solution phase, were then developed on a standard 8% polyacrylamide gel using standard procedures. The results are shown in the autoradiograph of Fig. 7 in which

| Lane | Explanation |
|---|---|
| 1 | Sequence produced on solid support bound material after washing with 2.5 ml of annealing mix |
| 2 | First 200 µl sequence produced |
| 3 | Second 200 µl sequence produced |
| 4 | Etc |
| 5 | " |
| 6 | " |
| 7 | " |
| 8 | " |

(continued)

| Lane | Explanation |
|---|---|
| 9 | " |
| 10 | " |
| 11 | " |
| 12 | " |

Lane 1 demonstrates that the target nucleic acid (viral DNA) was indeed bound to the support (as proven by the fact that the primer sequence (3) was able to anneal to the target nucleic acid and permit the sequencing reaction to take place). Lanes 1-10 also demonstrate that there is no cross-talk or smear from the E. coli DNA. Therefore the viral DNA had been selectively retained on the support. Additionally the support bound target DNA is stably held on the support. There is no steric hindrance of the Klenow fragment by the support itself since sequencing reactions have been performed on support bound DNA.

Lanes 2-12 demonstrate progressively decreasing amounts of target DNA (i.e. viral DNA) in the washings from the column. Therefore excess viral DNA had been added to the column and was not annealed to the oligonucleotide sequence (3). It is therefore possible to "tune" the binding capacity of the column to the concentration of target nucleic acid.

APPENDIX A

Animal viruses

Pig Parvovirus

Pigmycoplasma hypneumoniae

Herpes

Cytomegalovirus (CMV)

Epstein Barr

Simplex Herpesvirus

Papillomaviruses

Human Papilloma virus 6 (HPV)

.. .. .. 11

.. .. .. 16

.. .. .. 18

.. .. .. 33

Parvoviruses

Parvovirus B 19

Picornaviruses

Rhinovirus (Enterovirus)

Rhinovirus HRV 2-14

Hepatitus virus

Hepatitis A (HPV)

Hepatitis B

Hepatitis C

Hepatitis D

Retroviruses

Human immunodeficiency virus 1 (HIV I)

.. .. .. 2 (HIV II)

Human T cell lymphoblastic virus I (HTLV I)

.. .. .. .. II (HTVL II)

Bacteria

Legionella pneumophilia
Mycobacterium avium
bovis
fortuitum
tuberculosis
Mycoplasma pneumoniae
Escherichia coli toxin
Borrelia burgdorferi
Clamydia trachomatis
Salmonella Typhimurium
Staphylococcus Aureus
Clostridium Perfringens
Klebsiella Pneumoniae
Aeromonas Salmonicida
Mycobalterium Bovis

Parasites

Trypanosoma:
Trypanosoma brucei brucei
Trypanosoma cruzi
Trypanosoma congolense

Toxoplasma
Toxoplasma gondii

Plasmodia
Plasmodium falciparum
ovale
vivax
malariae

## Claims

1. A method of effecting a manipulation of a nucleic acid sequence comprising the steps of

(a) providing in a flow-through vessel a solid support system having covalently bonded thereto, single stranded oligonucleotide having a sequence of bases complementary to a portion of the sequence of a particular target nucleic acid having a greater number of bases than said oligonucleotide, said oligonucleotides being specific for said target nucleic acid,

(b) adding a source of single stranded target nucleic acid to the solid support system,

(c) hybridising the target nucleic acid to the oligonucleotide,

(d) effecting a flow through washing of the support after said hybridisation to remove any non-hybridised target nucleic acid in said support system thereby providing a pure sample of the target nucleic acid on the solid support system, and

(e) effecting on the target nucleic acid immobilised on the support in the flow through vessel a manipulation selected from copying of, denaturation of and/or hybridisation to said target nucleic acid.

**2.** A method as claimed in claim 1 wherein the flow through vessel is a column.

**3.** A method as claimed in claim 1 or 2 wherein the solid support system comprises silica.

**4.** A method as claimed in any one of claims 1 to 3 wherein the solid support system comprises particles.

**5.** A method as claimed in claim 4 wherein the particles are non-porous.

**6.** A method as claimed in claim 4 wherein the particles have a size of 100 to 200 μm (microns).

**7.** A method as claimed in any one of claims 1 to 6 wherein the oligonucleotides are covalently bonded to the solid support system by base stable covalent bonds.

**8.** A method as claimed in claim 7 wherein said bonds are stable for 2 hours in 38% $NH_4OH$ at 55°C.

**9.** A method as claimed in any one of claims 1 to 8 wherein the solid support system has a cross-linked siloxane matrix.

**10.** A method as claimed in any one of claims 1 to 9 wherein the flow through vessel has a size of 150-250 μl.

**11.** A method as claimed in any one of claims 1 to 10 wherein the manipulation comprises copying of the target nucleic acid sequence to produce a copy of at least part of said sequence.

**12.** A method as claimed in claim 11 wherein the 5' end of the oligonucleotide is covalently bonded to the support and said copying comprises extending the oligonucleotide in the 5' to 3' direction using the hybridised target nucleic acid as a template thereby producing an immobilised copy of the target covalently bonded to the support.

**13.** A method as claimed in claim 12 comprising the further steps of

(i) denaturing the target nucleic acid from said immobilised copy of the target nucleic acid covalently bonded to the support,

(ii) effecting a flow through wash of the solid support system to remove said target nucleic acid therefrom,

(iii) hybridising a primer to said immobilised copy of the target nucleic acid covalently bonded to the support, and

(iv) extending the primer in the 5' to 3' direction back towards said solid support using said copy of the target nucleic acid as a template thereby producing an extended primer product.

**14.** A method as claimed in claim 13 further comprising the steps of

(v) denaturing the extended primer product from said immobilised copy, and

(vi) collecting the extended primer product.

**15.** A method as claimed in claim 14 comprising repeating steps (i)-(vi) at least once.

**16.** A method as claimed in claim 13 wherein said primer added in step (iii) is labelled so as to produce labelled extended primer product and the method further comprises the step of detecting said labelled extended primer product.

**17.** A method as claimed in claim 11 wherein the 3' end of the oligonucleotide is covalently bonded to the support and the method comprises the step of hybridising a primer to the target nucleic acid, and said copying comprises extending said primer in the 5' to 3' direction back towards said support to produce an extended primer product.

**18.** A method as claimed in claim 17 further comprising the steps of denaturing the extended primer product from the target nucleic acid whilst leaving said target nucleic acid hybridised to the oligonucleotide.

**19.** A method as claimed in claim 17 which is a sequencing reaction comprising the steps of

(i) effecting said copying reaction with a mixture of the four deoxynucleotides dATP, dTTP, dGTP and dCTP together with a single one of the four dideoxynucleotides, ddATP, ddTTP, ddGTP and ddCTP.

(ii) denaturing and collecting the extended primer products whilst leaving the target nucleic acid hybridised to the oligonucleotide,

(iii) repeating steps (i) and (ii) in turn with each of the other three dideoxynucleotides, and

(iv) analysing the collected extended primer products to determine the sequence of the target nucleic acid.

**20.** A method as claimed in any one of claims 1 to 10 wherein said manipulation comprises denaturation of the target nucleic acid from said oligonucleotides.

**21.** A method as claimed in any one of claims 1 to 10 wherein said manipulation comprises hybridisation of a labelled probe to the target nucleic acid.

**22.** Apparatus for effecting a manipulation on a nucleic acid sequence comprising

a flow through vessel provided with a solid support system to which are covalently bonded, by base stable linkages, single stranded oligonucleotide having a sequence of bases complementary to a portion of the sequence of a particular target nucleic acid having a greater number of bases than said oligonucleotide, said oligonucleotide being specific for said target nucleic acid,

storage means for storing solutions removed from the vessel during washing or other procedures before the solutions are returned to the vessel,

detector means for detecting products of nucleic acid manipulations, and control means for diverting solutions into and out of the vessel, into waste or storage areas and to the detector means.

**23.** Apparatus as claimed in claim 22 wherein the flow through vessel is a column.

**24.** Apparatus as claimed in claim 22 or 23 wherein the solid support system comprises particles.

**25.** Apparatus as claimed in claim 24 wherein the particles have a size of 100 to 200 μm (microns).

**26.** Apparatus as claimed in claim 24 or 25 wherein the particles are non-porous.

**27.** Apparatus as claimed in claim 24 wherein the particles are of silica.

**28.** Apparatus as claimed in any one of claims 22 to 27 wherein the oligonculeotides are covalently bonded to the solid support by covalent bonds which are stable for 2 hours in 38% $NH_4OH$ at 55°C.

**29.** Apparatus as claimed in any one of claims 22 to 28 wherein the solid support system is provided with a cross-linked siloxane matrix to which said oligonucleotides are covalently bonded to provide said acid and base stable linkage of the oligonucleotides to the solid support system.

**30.** Apparatus as claimed in any one of claims 22 to 29 wherein the vessel has a volume of 150 to 250µl.

**31.** A flow through vessel having an inlet and an outlet and containing a solid support system having covalently bonded thereto, by base stable linkages, single stranded oligonucleotide having a sequence of bases complementary to a portion of the sequence of a particular target nucleic acid having a greater number of bases than said oligonucleotide, said oligonucleotide being specific for said target nucleic acid, said vessel having porous retaining elements to retain said particles in the vessel.

**32.** A vessel as claimed in claim 31 which is a column.

**33.** A vessel as claimed in claim 31 or 32 wherein the said solid support system comprises particles.

**34.** A vessel as claimed in claim 33 wherein the particles have a size of 100 to 200 µm (microns).

**35.** A vessel as claimed in claims 33 or 34 wherein the particles are non-porous.

**36.** A vessel as claimed in any one of claims 33 to 35 wherein the particles are of silica.

**37.** A vessel as claimed in any one of claims 31 to 36 wherein the oligonucleotides are covalently bonded to the solid support system by covalent bonds which are stable for 2 hours in 38% $NH_4OH$ at 55°C.

**38.** A vessel as claimed in any one of claims 31 to 37 wherein the particles are provided with a cross-linked siloxane matrix to which said oligonucleotides are covalently bonded to provide said acid base stable linkage of the oligonucleotides to said particles.

**39.** A vessel as claimed in any one of claims 32 to 38 wherein the vessel has a volume of 150 to 250µl.

**Patentansprüche**

**1.** Methode zur Ausführung einer Manipulation einer Nucleinsäuresequenz, wobei die Methode folgende Schritte umfaßt:

(a) Bereitstellen in einem Durchlaufgefäß eines festen Trägersystems, an das ein einzelsträngiges Oligonucleotid kovalent gebunden ist, das eine Sequenz von Basen aufweist, die einem Teil der Sequenz einer bestimmten, eine höhere Anzahl von Basen als das Oligonucleotid aufweisenden Zielnucleinsäure komplementär ist, wobei die Oligonucleotide für die Zielnucleinsäure spezifisch sind,

(b) Anlagern einer Quelle einer einzelsträngigen Zielnucleinsäure an das feste Trägersystem,

(c) Hybridisieren der Zielnucleinsäure an das Oligonucleotid,

(d) Bewirken eines Durchlaufwaschens des Trägers nach der Hybridisierung zur Entfernung jeglicher nichthybridisierter Zielnucleinsäure in dem Trägersystem, wobei eine reine Probe der Zielnucleinsäure an dem festen Trägersystem bereitgestellt wird, und

(e) Durchführen an der an dem Träger immobilisierten Zielnucleinsäure in den Durchlaufgefäß einer Manipulation, die unter dem Kopieren der, der Denaturierung der und/oder der Hybridisierung an die Zielnucleinsäure ausgewählt wird.

**2.** Methode nach Anspruch 1, bei der das Durchlaufgefäß eine Säule ist.

3. Methode nach Anspruch 1 oder 2, bei der das feste Trägersystem Siliciumdioxid umfaßt.

4. Methode nach einem der Ansprüche 1 bis 3, bei der das feste Trägersystem Teilchen umfaßt.

5. Methode nach Anspruch 4, bei der die Teilchen nicht porenhaltig sind.

6. Methode nach Anspruch 4, bei der die Teilchen eine Größe von 100 bis 200 μm (Mikrometern) aufweisen.

7. Methode nach einem der Ansprüche 1 bis 6, bei der die Oligonucleotide durch basenstabile kovalente Bindungen kovalent an das feste Trägersystem gebunden sind.

8. Methode nach Anspruch 7, bei der die Bindungen 2 Stunden in 38%igem $NH_4OH$ bei 55°C beständig sind.

9. Methode nach einem der Ansprüche 1 bis 8, bei der das feste Trägersystem eine vernetzte Siloxanmatrix aufweist.

10. Methode nach einem der Ansprüche 1 bis 9, bei der das Durchlaufgefäß eine Größe von 150-250 μl aufweist.

11. Methode nach einem der Ansprüche 1 bis 10, bei der die Manipulation das Kopieren der Zielnucleinsäure-Sequenz unter Bildung einer Kopie mindestens eines Teils der Sequenz umfaßt.

12. Methode nach Anspruch 11, bei der das 5'-Ende des Oligonucleotids kovalent an den Träger gebunden ist und das Kopieren das Verlängern des Oligonucleotids in 5'→3'-Richtung unter Zuhilfenahme der hybridisierten Zielnucleinsäure als Matrize umfaßt, wobei eine immobilisierte Kopie der kovalent an den Träger gebundenen Zielnucleinsäure gebildet wird.

13. Methode nach Anspruch 12, die des weiteren folgende Schritte umfaßt:

    (i) das Denaturieren der Zielnucleinsäure von der immobilisierten Kopie der kovalent an den Träger gebundenen Zielnucleinsäure,

    (ii) das Bewirken eines Durchlaufwaschens des festen Trägersystems zur Entfernung der Zielnucleinsäure von diesem,

    (iii) das Hybridisieren eines Primers an die immobilisierte Kopie der kovalent an den Träger gebundenen Zielnucleinsäure, und

    (iv) das Verlängern des Primers in der 5'→3'-Richtung zurück auf den festen Träger zu unter Zuhilfenahme der Kopie der Zielnucleinsäure als Matrize unter Bildung eines verlängerten Primerprodukts.

14. Methode nach Anspruch 13, die des weiteren folgende Schritte umfaßt:

    (v) das Denaturieren des verlängerten Primerprodukts von der immobilisierten Kopie und

    (vi) das Einsammeln des verlängerten Primerprodukts.

15. Methode nach Anspruch 14, die das mindestens einmalige Wiederholen der Schritte (i) bis (vi) umfaßt.

16. Methode nach Anspruch 13, bei der der in Schritt (iii) angelagerten Primer markiert wird unter Bildung eines markierten verlängerten Primerprodukts und die Methode des weiteren den Schritt des Erfassens des markierten verlängerten Primerprodukts umfaßt.

17. Methode nach Anspruch 11, bei der das 3'-Ende des Oligonucleotids kovalent an den Träger gebunden ist und die Methode den Schritt des Hybridisierens eines Primers an die Zielnucleinsäure und das Kopieren das Verlängern des Primers in der 5'→3'-Richtung zurück aufden Träger zu unter Bildung eines verlängerten Primerprodukts umfaßt.

18. Methode nach Anspruch 17, die des weiteren die Schritte des Denaturierens des verlängerten Primerprodukts von der Zielnueleinsäure umfaßt, während die Zielnueleinsäure an das Oligonucleotid hybridisiert bleibt.

**19.** Methode nach Anspruch 17, bei der es sich um eine Sequenzierreaktion handelt, die folgende Schritte umfaßt:

(i) Ausführen der Kopierreaktion mit einer Mischung der vier Deoxynucleotide dATP, dTTP, dGTP und dCTP in Verbindung mit einem einzigen der vier Dideoxynucleotide ddATP, ddTTP, ddGTP und ddCTP,

(ii) Denaturieren und Einsammeln der verlängerten Primerprodukte, während die Zielnueleinsäure an das Oligonucleotid hybridisiert bleibt,

(iii) Wiederholen der Schritte (i) und (ii) nacheinander mit jedem der drei anderen Dideoxynucleotide und

(iv) Analysieren der eingesammelten verlängerten Primerprodukte zur Bestimmung der Sequenz der Zielnucleinsäure.

**20.** Methode nach einem der Ansprüche 1 bis 10, bei der die Manipulation das Denaturieren der Zielnueleinsäure von den Oligonucleotiden umfaßt.

**21.** Methode nach einem der Ansprüche 1 bis 10, bei der die Manipulation das Hybridisieren einer markierten Sonde an die Zielnucleinsäure umfaßt.

**22.** Einrichtung für die Ausführung einer Manipulation an einer Nucleinsäuresequenz, die folgendes umfaßt:

ein Durchlaufgefäß, das mit einem festen Trägersystem ausgestattet ist, an das durch basenstabile Verknüpfungen ein einzelsträngiges Oligonucleotid kovalent gebunden ist, das eine Sequenz von Basen aufweist, die einem Teil der Sequenz einer bestimmten, eine höhere Anzahl von Basen als das Oligonucleotid aufweisenden Zielnucleinsäure komplementär ist, wobei das Oligonucleotid für die Zielnucleinsäure spezifisch ist,

eine Lagervorrichtung für das Lagern von Lösungen, die während des Waschens oder anderen Vorgängen aus dem Gefäß entfernt worden sind, bevor sie in das Gefäß zurückgegeben werden,

eine Detektorvorrichtung für das Erfassen von Nucleinsäuremanipulationsprodukten und eine Regelvorrichtung für das Umleiten von Lösungen in das und aus dem Gefäß, in Abfallstoff- oder Lagerbereiche und zu der Detektorvorrichtung.

**23.** Einrichtung nach Anspruch 22, bei der das Durchlaufgefäß eine Säule ist.

**24.** Einrichtung nach den Ansprüchen 22 oder 23, bei der das feste Trägersystem Teilchen umfaßt.

**25.** Einrichtung nach Anspruch 24, bei der die Teilchen eine Größe von 100 bis 200 µm (Mikrometern) aufweisen.

**26.** Einrichtung nach Anspruch 24 oder 25, bei der die Teilchen nicht porenhaltig sind.

**27.** Einrichtung nach Anspruch 24, bei der die Teilchen aus Siliciumdioxid bestehen.

**28.** Einrichtung nach einem der Ansprüche 22 bis 27, bei der die Oligonucleotide durch kovalente Bindungen kovalent an den festen Träger gebunden sind, wobei die Bindungen 2 Stunden in 38%igem $NH_4OH$ bei 55°C beständig sind.

**29.** Einrichtung nach einem der Ansprüche 22 bis 28, bei der das feste Trägersystem mit einer vernetzten Siloxanmatrix ausgestattet ist, an die die Oligonucleotide kovalent gebunden sind, um die säure- und basenstabile Verknüpfungen der Oligonucleotide an das feste Trägersystem bereitzustellen.

**30.** Einrichtung nach einem der Ansprüche 22 bis 29, bei der das Gefäß ein Volumen von 150 bis 250 µl aufweist.

**31.** Durchlaufgefäß, das einen Einlaß und einen Auslaß aufweist und ein festes Trägersystem enthält, an das ein einzelsträngiges Oligonucleotid durch basenstabile Verknüpfungen kovalent gebunden ist, das eine Sequenz von Basen aufweist, die einem Teil der Sequenz einer bestimmten, eine höhere Anzahl von Basen als das Oligonucleotid aufweisenden Zielnucleinsäure komplementär ist, wobei das Oligonucleotid für die Zielnucleinsäure spezifisch ist, wobei das Gefäß porenhaltige Rückhalteelemente aufweist zum Zurückhalten der Teilchen in dem Gefäß.

**32.** Gefäß nach Anspruch 31, das eine Säule ist.

**33.** Gefäß nach Anspruch 31 oder 32, bei dem das feste Trägersystem Teilchen umfaßt.

**34.** Gefäß nach Anspruch 33, bei dem die Teilchen eine Größe von 100 bis 200 µm (Mikrometern) aufweisen.

**35.** Gefäß nach den Ansprüchen 33 oder 34, bei dem die Teilchen nicht porenhaltig sind.

**36.** Gefäß nach einem der Ansprüche 33 bis 35, bei dem die Teilchen aus Siliciumdioxid bestehen.

**37.** Gefäß nach einem der Ansprüche 31 bis 36, bei dem die Oligonucleotide durch kovalente Bindungen kovalent an das feste Trägersystem gebunden sind, wobei die Bindungen 2 Stunden in 38%igem $NH_4OH$ bei 55°C beständig sind.

**38.** Gefäß nach einem der Ansprüche 31 bis 37, bei dem die Teilchen mit einer vernetzten Siloxanmatrix ausgestattet sind, an die die Oligonucleotide kovalent gebunden sind, um die säure- und basenstabilen Verknüpfungen der Oligonucleotide an die Teilchen bereitzustellen.

**39.** Gefäß nach einem der Ansprüche 32 bis 38, bei dem das Gefäß ein Volumen von 150 bis 250 µl aufweist.

**Revendications**

**1.** Méthode d'exécution d'une manipulation d'une séquence d'acides nucléiques comprenant les étapes consistant

a) à prévoir, dans un récipient de circulation, un système de support solide possédant des oligonucléotides à brin unique fixés à ce dernier par liaisons covalentes, ayant une séquence de bases complémentaire à une portion de la séquence d'un acide nucléique cible particulier, ayant un nombre de bases supérieur à celui dudit oligonucléotide, lesdits oligonucléotides étant spécifiques audit acide nucléique cible,

b) à ajouter une source d'acide nucléique cible à brin unique au système de support solide,

c) à procéder à l'hybridation de l'acide nucléique cible à l'oligonucléotide,

d) à exécuter un lavage par circulation du support après ladite hybridation pour éliminer tout acide nucléique cible non hybridé dudit système de support, en fournissant ainsi un échantillon pur de l'acide nucléique cible sur le système de support solide, et

e) à effectuer, sur l'acide nucléique cible immobilisé sur le support, dans le récipient de circulation, une manipulation choisie parmi la copie, la dénaturation et/ou l'hybridation dudit acide nucléique cible.

**2.** Méthode selon la revendication 1, dans laquelle le récipient de circulation est une colonne.

**3.** Méthode selon la revendication 1 ou 2, dans laquelle le système de support solide comprend de la silice.

**4.** Méthode selon l'une quelconque des revendications 1 à 3, dans laquelle le système de support solide comprend des particules.

**5.** Méthode selon la revendication 4, dans laquelle les particules sont non poreuses.

**6.** Méthode selon la revendication 4, dans laquelle les particules ont une grandeur de 100 à 200 µm (microns).

**7.** Méthode selon l'une quelconque des revendications 1 à 6, dans laquelle les oligonucléotides sont liés au système de support solide par des liaisons covalentes stables vis-à-vis des bases.

**8.** Méthode selon la revendication 7, dans laquelle lesdites liaisons sont stables pendant 2 heures dans du $NH_4OH$ à 38 % à 55 °C.

**9.** Méthode selon l'une quelconque des revendications 1 à 8, dans laquelle le système de support solide possède une

matrice de siloxane réticulée.

**10.** Méthode selon l'une quelconque des revendications 1 à 9, dans laquelle le récipient de circulation a une taille de 150-250 µl.

**11.** Méthode selon l'une quelconque des revendications 1 à 10, dans laquelle la manipulation comprend la copie de ladite séquence d'acides nucléiques cible en vue de la production d'une copie d'au moins une partie de ladite séquence.

**12.** Méthode selon la revendication 11, dans laquelle l'extrémité 5' de l'oligonucléotide est liée par des liaisons covalentes au support et ladite copie comprend l'extension de l'oligonucléotide dans la direction 5' à 3', par utilisation de l'acide nucléique cible hybridé en tant que matrice, en produisant ainsi une copie immobilisée de la cible liée par liaisons covalentes au support.

**13.** Méthode selon la revendication 12, comprenant les étapes supplémentaires consistant

(i) à dénaturer l'acide nucléique cible de ladite copie immobilisée de l'acide nucléique cible, lié par liaisons covalentes au support,

(ii) à effectuer un lavage par circulation du système de support solide pour en éliminer ledit acide nucléique cible,

(iii) à procéder à l'hybridation d'une amorce sur ladite copie immobilisée de l'acide nucléique cible liée par liaisons covalentes au support, et

(iv) d'étendre l'amorce dans la direction 5' à 3' en retour vers ledit support solide, par utilisation de ladite copie de l'acide nucléique cible en tant que matrice, en produisant ainsi un produit d'amorce étendue.

**14.** Méthode selon la revendication 13, comprenant en outre les étapes consistant

(v) à dénaturer le produit d'amorce étendue de ladite copie immobilisée et

(vi) à recueillir le produit d'amorce étendue.

**15.** Méthode selon la revendication 14, comprenant la répétition des étapes (i)-(vi) à au moins une reprise.

**16.** Méthode selon la revendication 13, dans laquelle ladite amorce ajoutée dans l'étape (iii) est marquée de manière à produire un produit d'amorce étendue marquée et la méthode comprend en outre l'étape de détection dudit produit d'amorce étendue marquée.

**17.** Méthode selon la revendication 11, dans laquelle l'extrémité 3' de l'oligonucléotide est liée par des liaisons covalentes au support et la méthode comprend l'étape d'hybridation d'une amorce à l'acide nucléique cible, et ladite copie comprend l'extension de ladite amorce dans la direction 5' à 3' en retour vers ledit support pour produire un produit d'amorce étendue.

**18.** Méthode selon la revendication 17, comprenant en outre les étapes de dénaturation du produit d'amorce étendue à partir de l'acide nucléique cible tout en laissant inchangé ledit acide nucléique cible hybridé à l'oligonucléotide.

**19.** Méthode selon la revendication 17, qui est une réaction de séquençage comprenant les étapes consistant

(i) à effectuer ladite réaction de copie avec un mélange des quatre déoxynucléotides dATP, dTTP, dGTP et dCTP, conjointement à un seul des quatre didéoxynucléotides ddATP, ddTTP, ddGTP et ddCTP,

(ii) à dénaturer et à recueillir les produits d'amorce étendue tout en laissant inchangé l'acide nucléique cible hybridé à l'oligonucléotide,

(iii) à répéter les étapes (i) et (ii) tour à tour avec chacun des trois autres didéoxynucléotides, et

(iv) à analyser les produits d'amorce étendue recueillis pour déterminer la séquence de l'acide nucléique cible.

**20.** Méthode selon l'une quelconque des revendications 1 à 10, dans laquelle ladite manipulation comprend la dénaturation de l'acide nucléique cible desdits oligonucléotides.

**21.** Méthode selon l'une quelconque des revendications 1 à 10, dans laquelle ladite manipulation comprend l'hybridation d'un échantillon marqué à l'acide nucléique cible.

**22.** Appareil destiné à effectuer une manipulation sur une séquence d'acide nucléique comprenant

un récipient de circulation muni d'un système de support solide auquel sont liés par des liaisons covalentes stables vis-à-vis des bases un oligonucléotide à brin unique ayant une séquence de bases complémentaire à une portion de la séquence d'un acide nucléique cible particulier, ayant un nombre de bases supérieur à celui dudit oligonucléotide, ledit oligonucléotide étant spécifique audit acide nucléique cible,

des moyens de stockage pour l'entreposage des solutions retirées du récipient pendant le lavage ou pendant d'autres procédures avant le retour des solutions au récipient,

des moyens de détection destinés à la détection de produits en provenance de manipulation d'acides nucléiques et des moyens de contrôle en vue de la déviation des solutions dans et hors du récipient, vers des zones de mise au rebut ou de stockage et vers les moyens de détection.

**23.** Appareil selon la revendication 22, dans lequel le récipient de circulation est une colonne.

**24.** Appareil selon la revendication 22 ou 23, dans lequel le système de support solide comprend des particules.

**25.** Appareil selon la revendication 24, dans lequel les particules ont une grandeur de 100 à 200 $\mu$m (microns).

**26.** Appareil selon la revendication 24 ou 25, dans lequel les particules sont non poreuses.

**27.** Appareil selon la revendication 24, dans lequel les particules sont faites de silice.

**28.** Appareil selon l'une quelconque des revendications 22 à 27, dans lequel les oligonucléotides sont liés au support solide par des liaisons covalentes qui sont stables pendant 2 heures dans du $NH_4OH$ à 38 % à 55°C.

**29.** Appareil selon l'une quelconque des revendications 22 à 28, dans lequel le système de support solide est muni d'une matrice de siloxane réticulée à laquelle lesdits oligonucléotides sont liés par des liaisons covalentes pour fournir ladite liaison stable vis-à-vis des acides et des bases des oligonucléotides au système de support solide.

**30.** Appareil selon l'une quelconque des revendications 22 à 29, dans lequel le récipient a un volume de 150 à 250 $\mu$l.

**31.** Récipient de circulation ayant une entrée et une sortie et contenant un système de support solide possédant, lié par des liaisons stables vis-à-vis des bases, un oligonucléotide à brin unique, ayant une séquence de bases complémentaire à une portion de la séquence d'un acide nucléique cible particulier, ayant un nombre de bases supérieur à celui dudit oligonucléotide, ledit oligonucléotide étant spécifique audit acide nucléique cible, ledit récipient ayant des éléments poreux de retenue pour retenir lesdites particules dans le récipient.

**32.** Récipient selon la revendication 31, qui est une colonne.

**33.** Récipient selon la revendication 31 ou 32, dans lequel ledit système de support solide comprend des particules.

**34.** Récipient selon la revendication 33, dans lequel les particules ont une grandeur de 100 à 200 $\mu$m (microns).

**35.** Récipient selon les revendications 33 ou 34, dans lequel les particules sont non poreuses.

**36.** Récipient selon l'une quelconque des revendications 33 à 35, dans lequel les particules sont faites de silice.

**37.** Récipient selon l'une quelconque des revendications 31 à 36, dans lequel les oligonucléotides sont liés au système

de support solide par des liaisons covalentes qui sont stables pendant 2 heures dans du $NH_4OH$ à 38% à 55°C.

**38.** Récipient selon l'une quelconque des revendications 31 à 37, dans lequel les particules sont munies d'une matrice de siloxane réticulée à laquelle lesdits oligonucléotides sont liés par des liaisons covalentes pour fournir ladite liaison stable vis-à-vis des acides et des bases des oligonucléotides auxdites particules.

**39.** Récipient selon l'une quelconque des revendications 32 à 38, dans lequel le récipient a un volume de 150 à 250 µl.

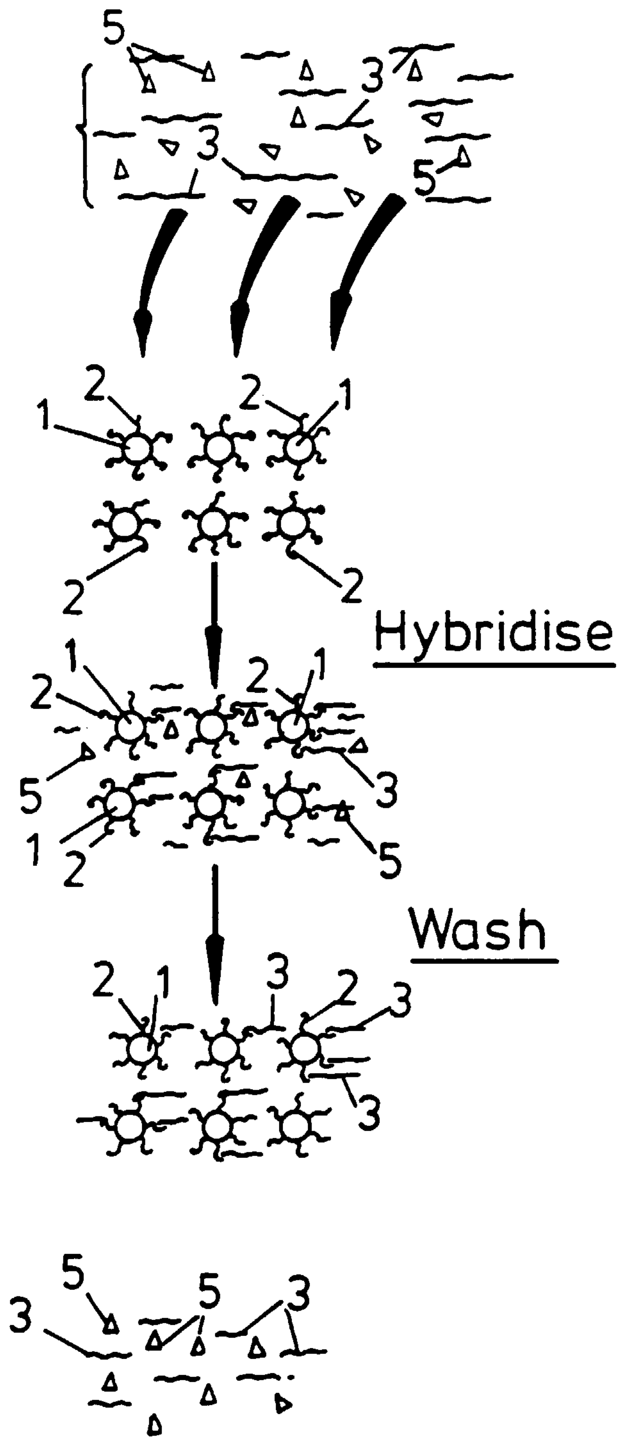


FIG. 1

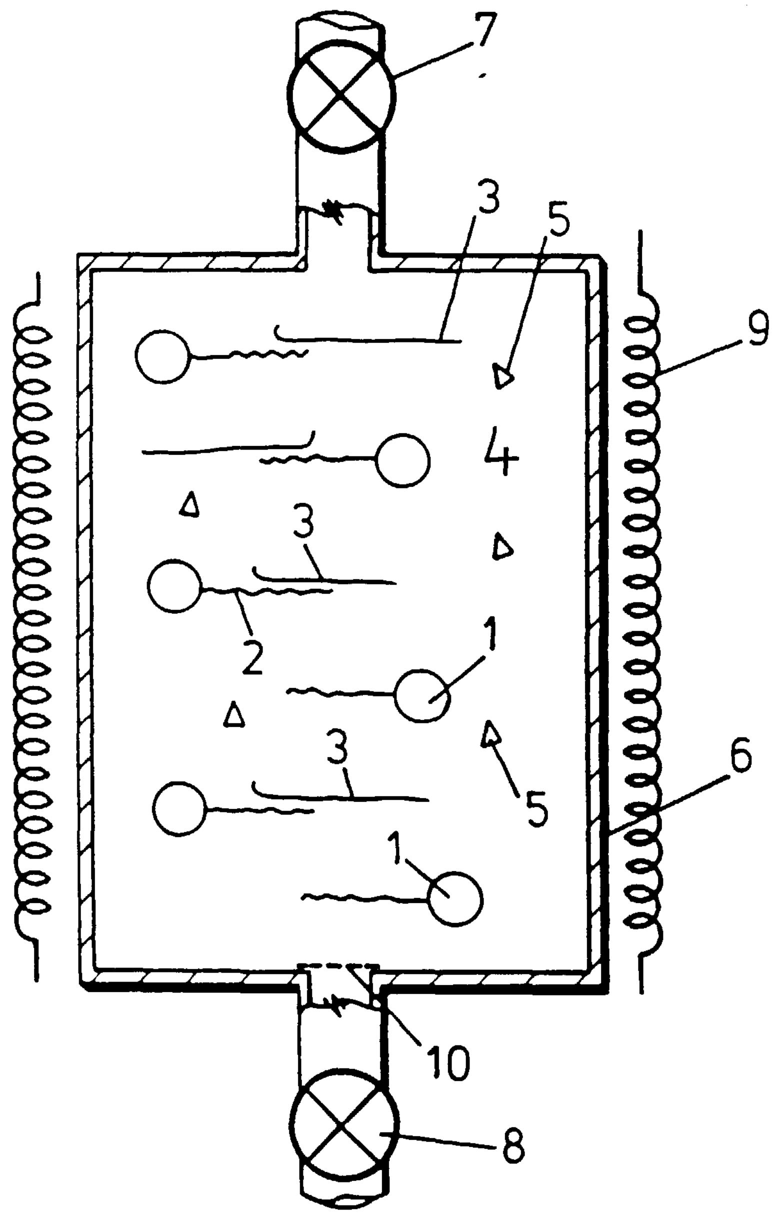


FIG. 2

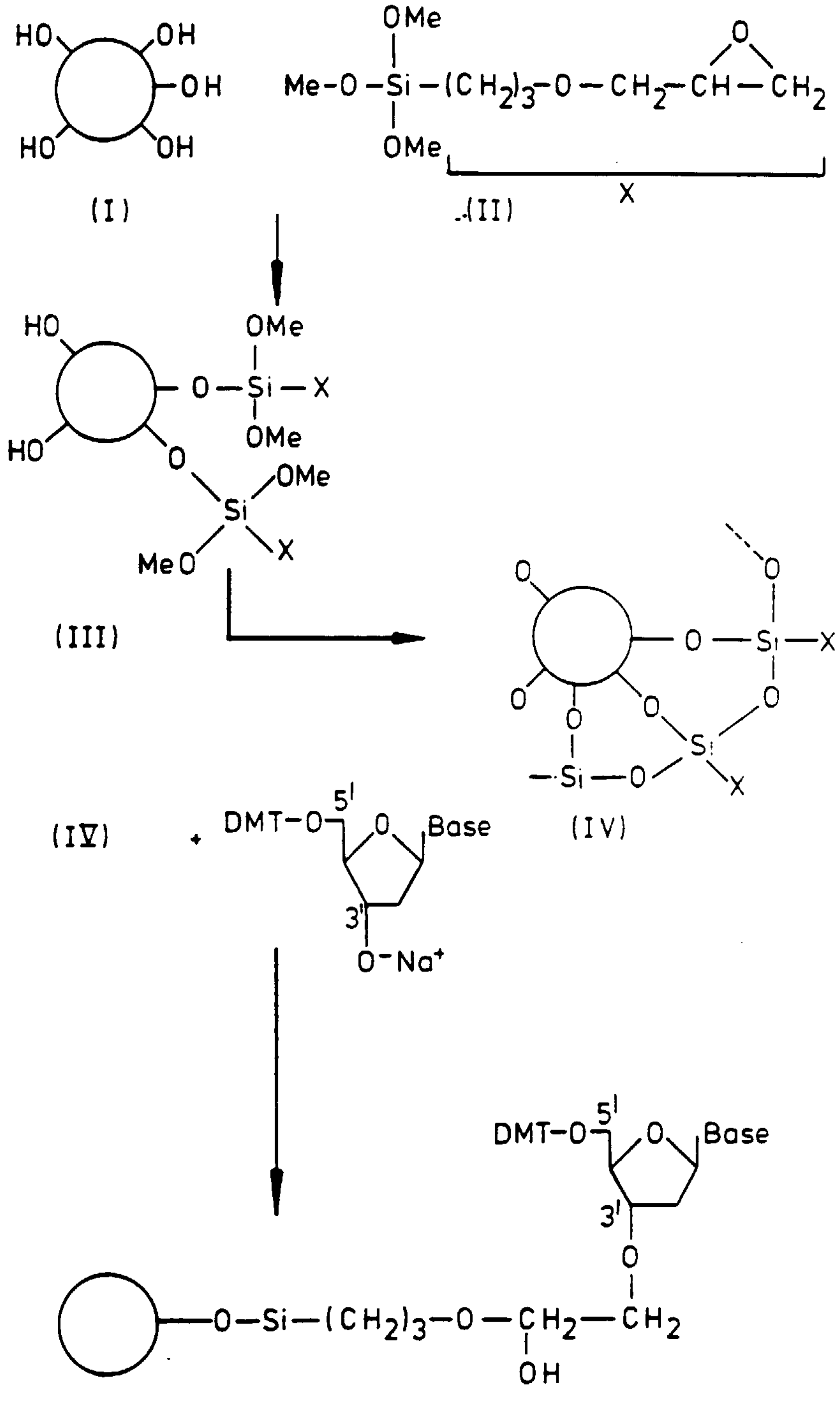

(I)

(II)

(III)

(IV) +

(IV)

(V)

FIG. 3

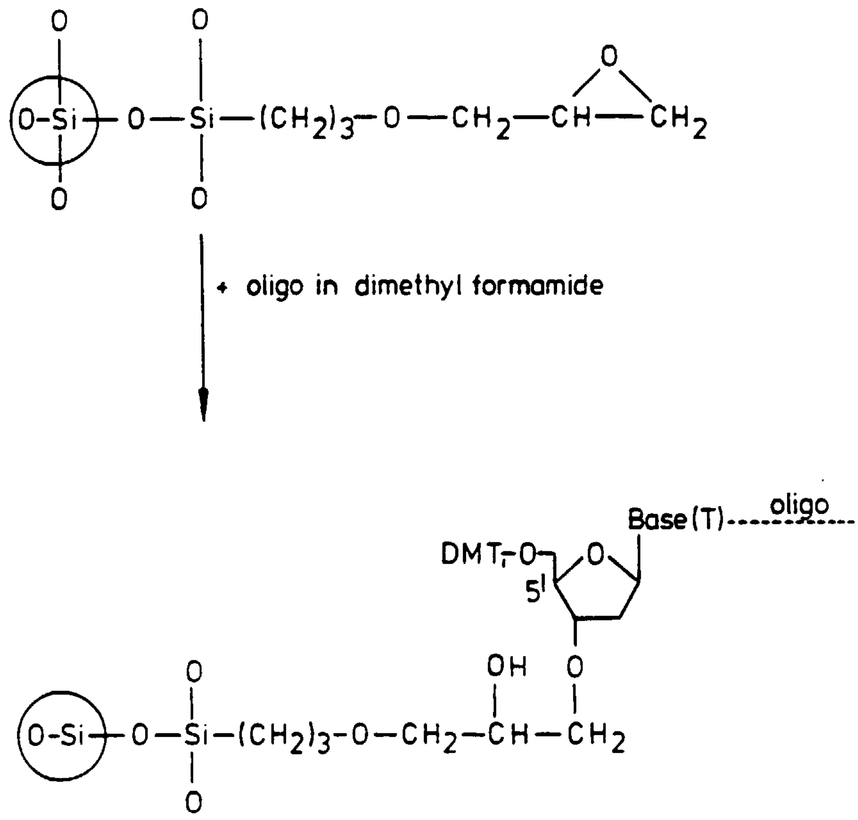


FIG. 4a

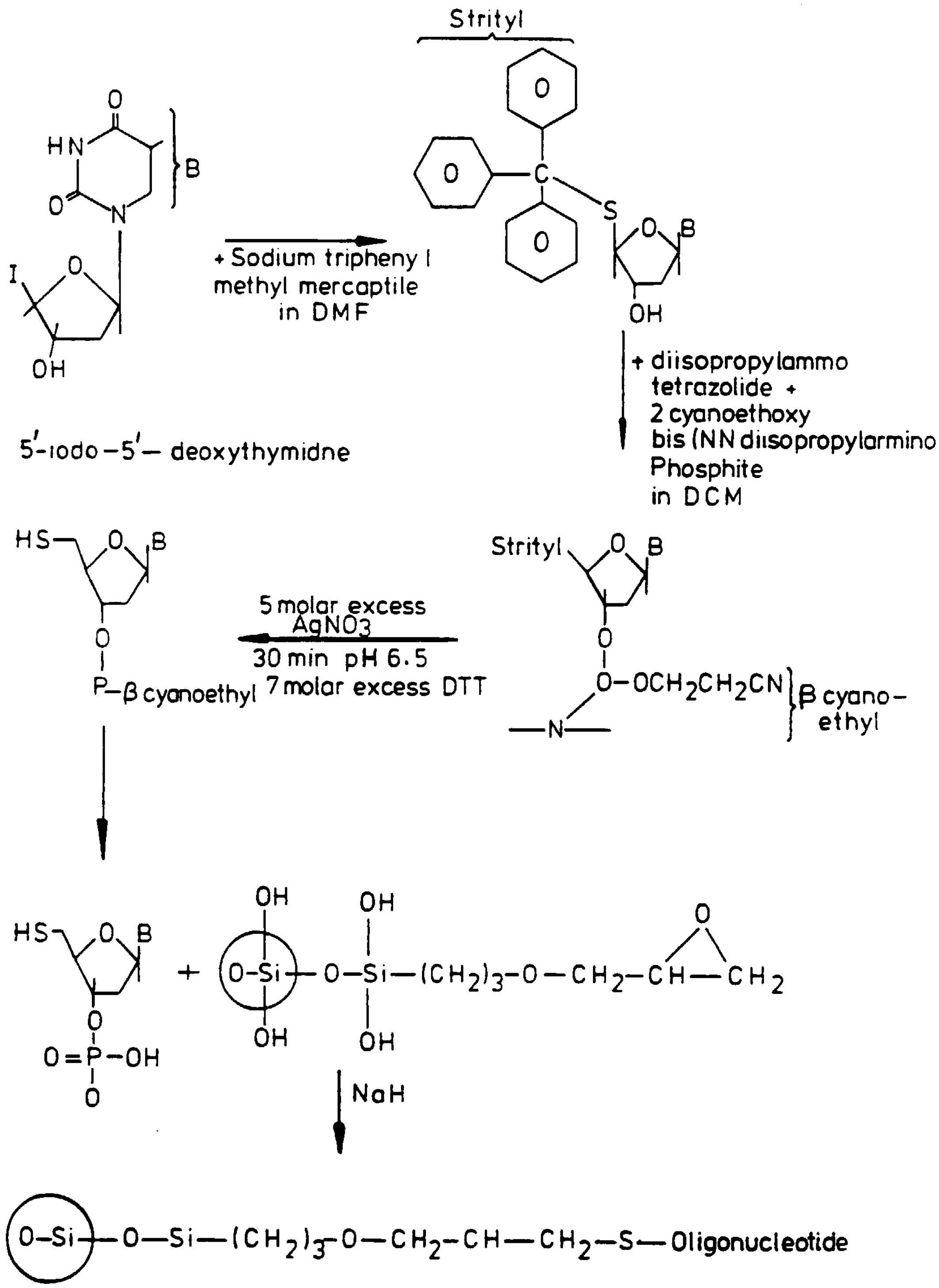


5'-iodo-5'- deoxythymidne

FIG. 4b

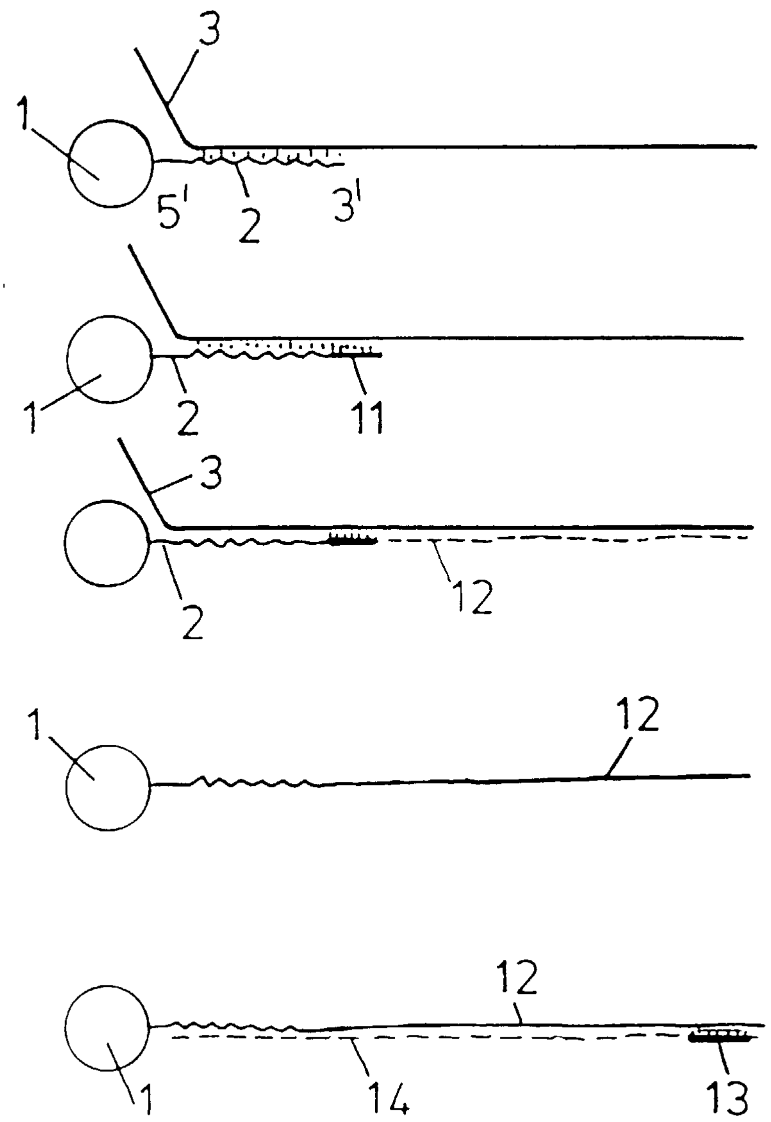


FIG.5a

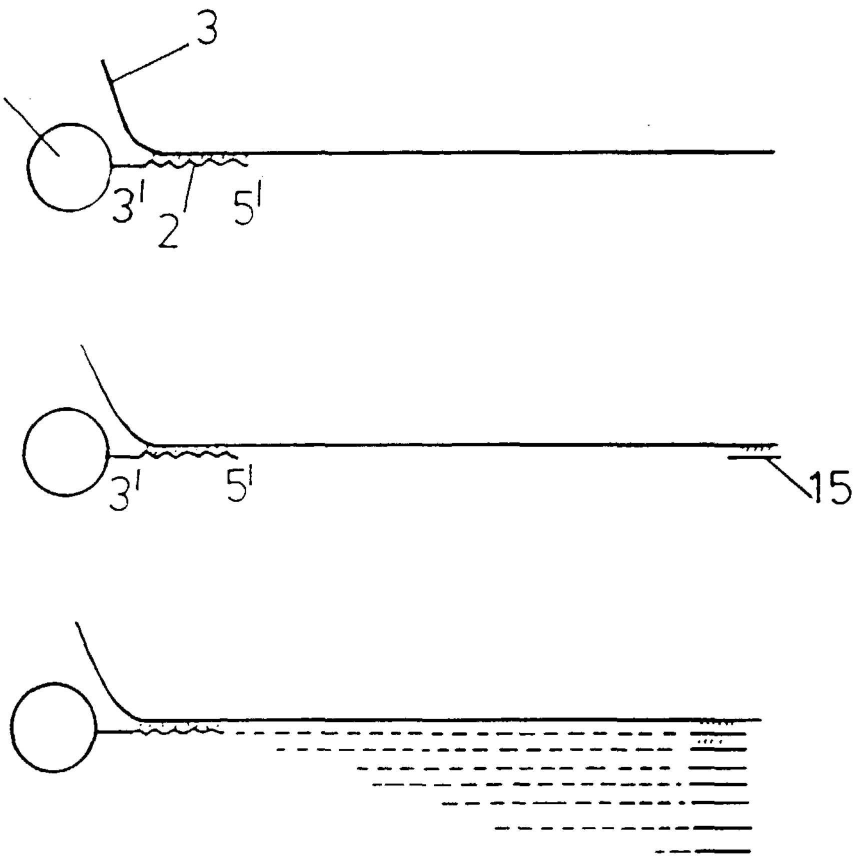


FIG.5b

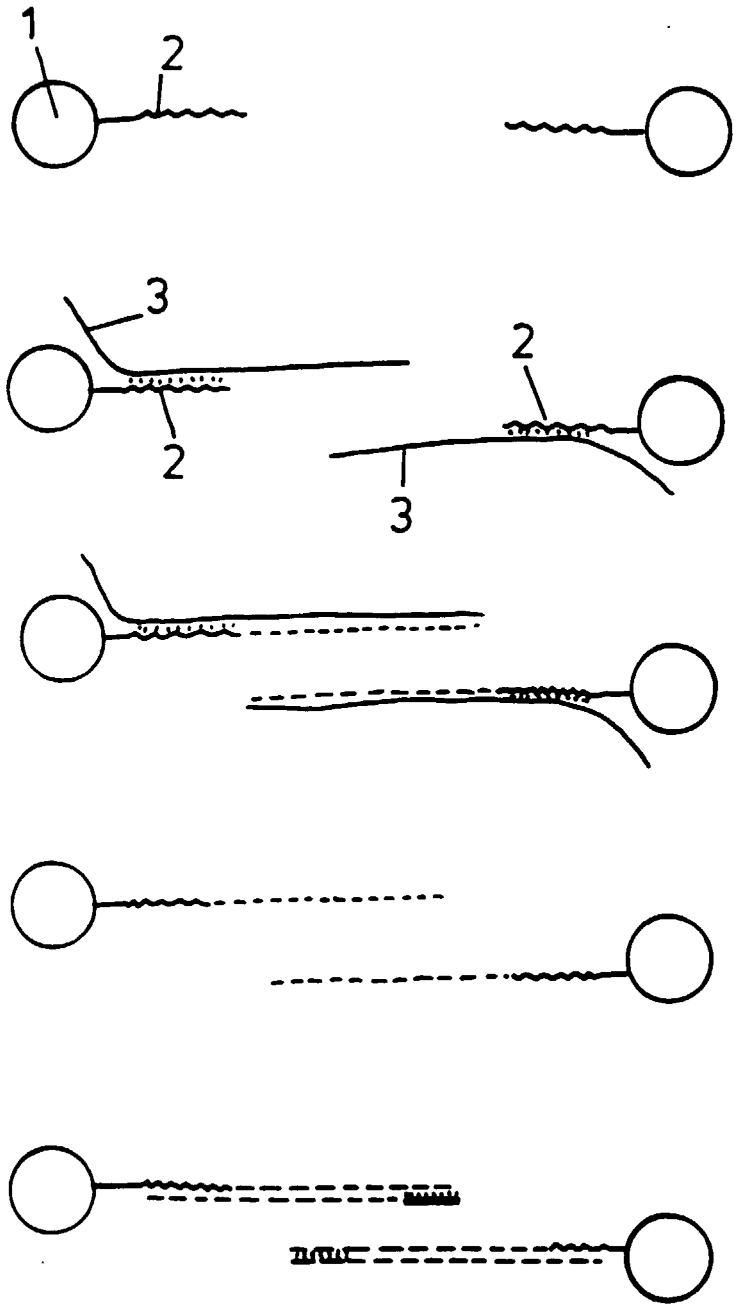


FIG. 5c

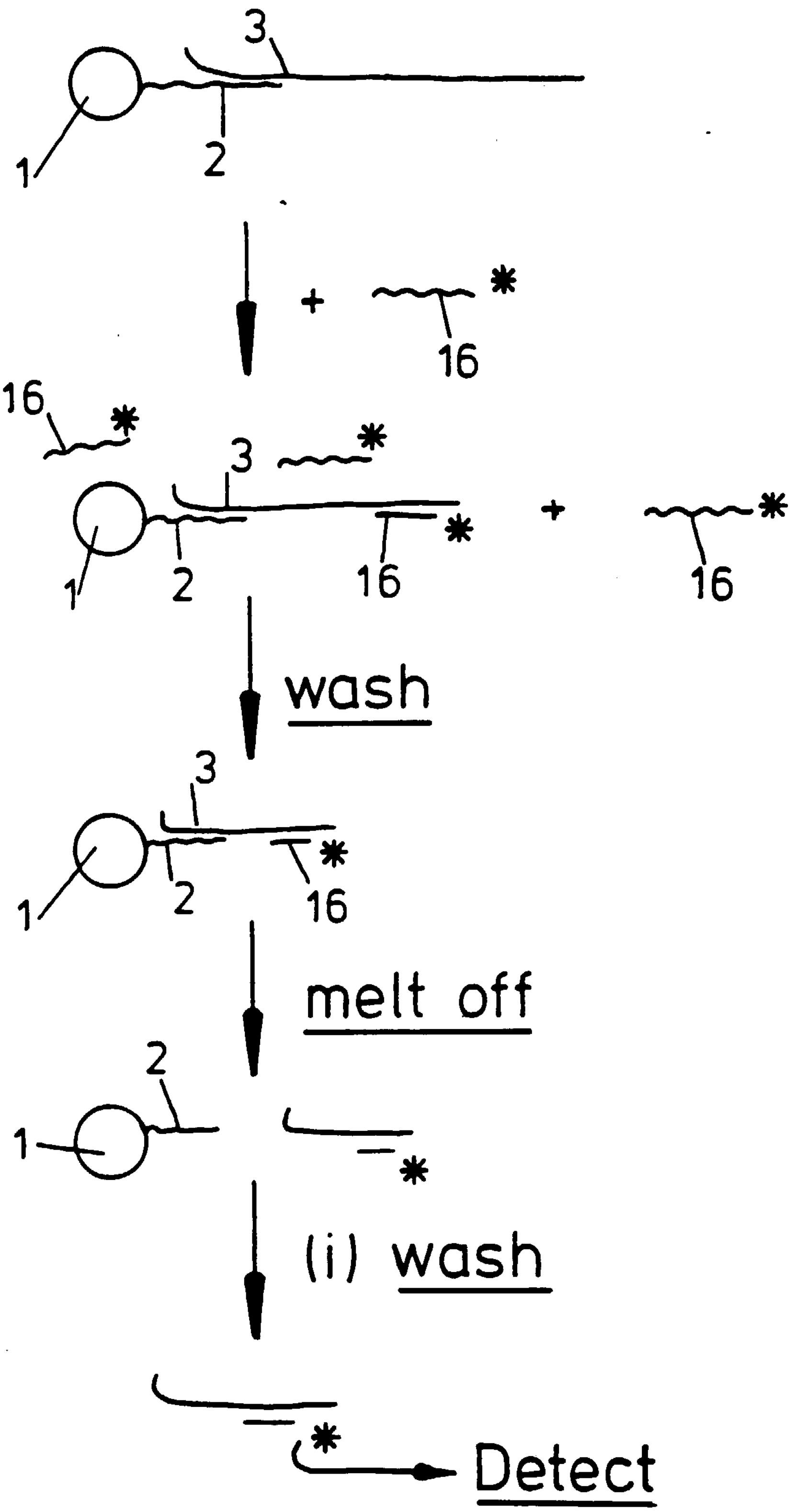


FIG. 5d

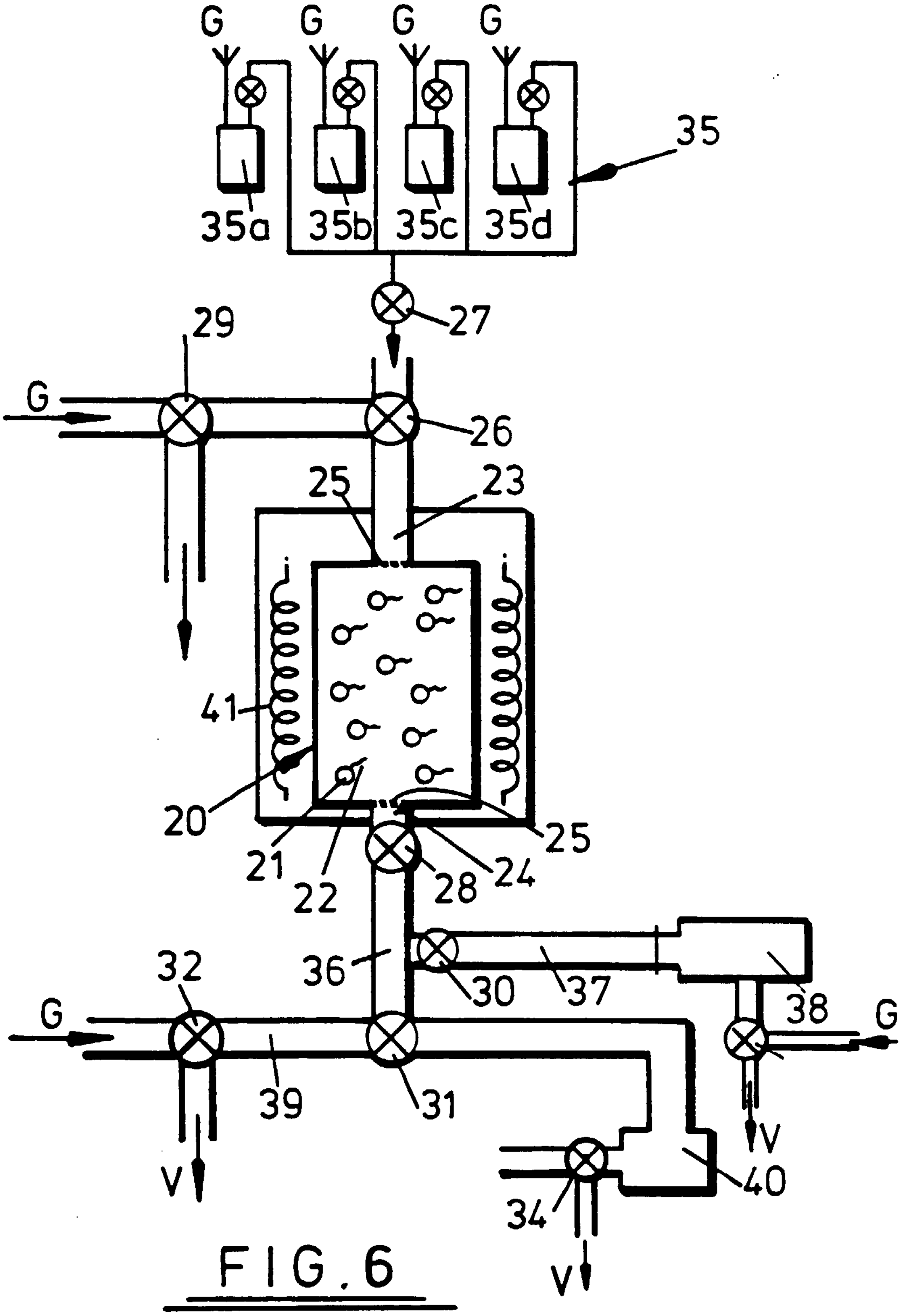
G
G
G
G
35
35a
35b
35c
35d
27
29
G
26
25
23
41
20
21
22
28
24
25
G
32
36
30
37
38
G
39
31
V
V
34
40
V

FIG.6

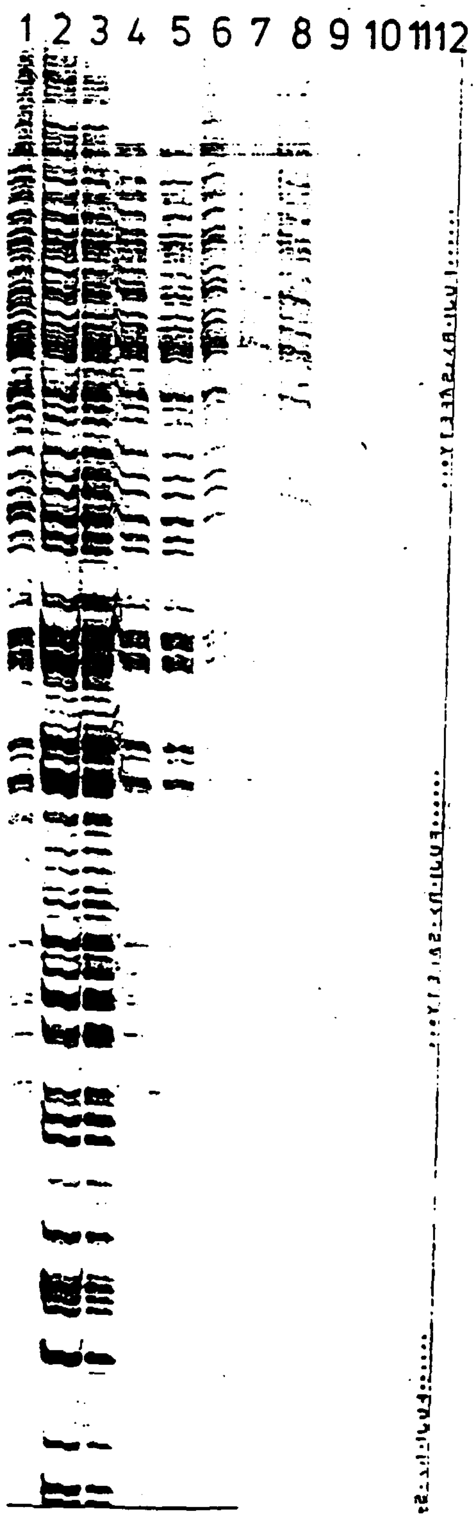


FIG. 7